(19) Europäisches Patentamt
European Patent Office
Office européen des brevets

(11) Veröffentlichungsnummer: **0 006 600**
**B1**

(12) # EUROPÄISCHE PATENTSCHRIFT

(45) Veröffentlichungstag der Patentschrift: **16.09.81**

(51) Int. Cl.³: **C 07 C 69/74,** A 01 N 53/00

(21) Anmeldenummer: **79102086.0**

(22) Anmeldetag: **25.06.79**

(54) **Tetrahalogenäthylcyclopropankarbonsäureester, Verfahren zu ihrer Herstellung und ihre Verwendung in der Schädlingsbekämpfung.**

(30) Priorität: **27.06.78 CH 6992/78**
**15.01.79 CH 356/79**
**16.05.79 CH 4553/79**

(43) Veröffentlichungstag der Anmeldung: **09.01.80 Patentblatt 80/1**

(45) Bekanntmachung des Hinweises auf die Patenterteilung: **16.09.81 Patentblatt 81/37**

(84) Benannte Vertragsstaaten: **BE CH DE FR GB IT NL**

(56) Entgegenhaltungen:
**DE-A-2 805 193**
**DE-A-2 805 312**

(73) Patentinhaber: **CIBA-GEIGY AG, Patentabteilung Postfach, CH-4002 Basel (CH)**

(72) Erfinder: **Farooq, Saleem, Dr., Im Schalengarten 2, CH-4107 Ettingen (CH)**
Erfinder: **Ackermann, Peter, Dr., Reichensteinerstrasse 12, CH-4153 Reinach (CH)**
Erfinder: **Drabek, Jozef, Dr., Benkenstrasse 12, CH-4104 Oberwil (CH)**
Erfinder: **Gsell, Laurenz, Dr., Maiengasse 56, CH-4056 Basel (CH)**
Erfinder: **Kristiansen, Odd, Dr., Delligrabenstrasse 7, CH-4313 Möhlin (CH)**
Erfinder: **Wehrli, Rudolf, Dr., Dianastrasse 2, CH-4310 Rheinfelden (CH)**

(74) Vertreter: **Zumstein sen., Fritz, Dr. et al, Bräuhausstrasse 4, D-8000 München 2 (DE)**

**EP 0 006 600 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Tetrahalogenäthylcyclopropankarbonsäureester, Verfahren zu ihrer Herstellung und ihre Verwendung in der Schädlingsbekämpfung

Die vorliegende Erfindung betrifft Tetrahalogenäthylcyclopropankarbonsäureester, Verfahren zu ihrer Herstellung und ihre Verwendung in der Schädlingsbekämpfung.

Die Cyclopropankarbonsäureester haben die Formel

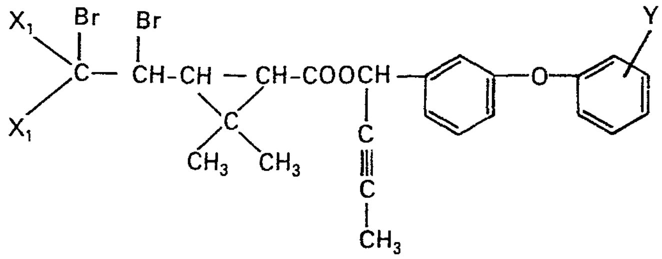

worin $X_1$ Fluor, Chlor oder Brom und Y Wasserstoff, Methyl, Fluor, Chlor, Brom oder Jod bedeuten.

Wegen ihrer Wirkung von Bedeutung sind Verbindungen der Formel I, worin $X_1$ Fluor, Chlor oder Brom und Y Wasserstoff, p-Methyl, p-Fluor, p-Chlor oder p-Brom bedeuten.

Insbesondere hervorzuheben sind aber Verbindungen der Formel I, worin $X_1$ Fluor, Chlor oder Brom und Y Wasserstoff bedeuten.

Die Verbindungen der Formel I werden nach an sich bekannten Methoden z.B. wie folgt hergestellt:

1) 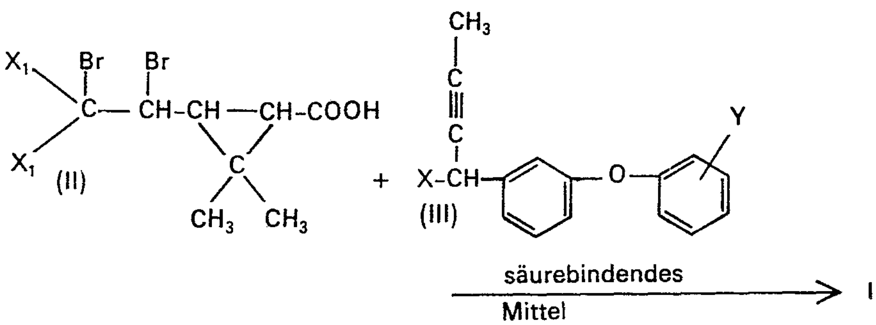


2) 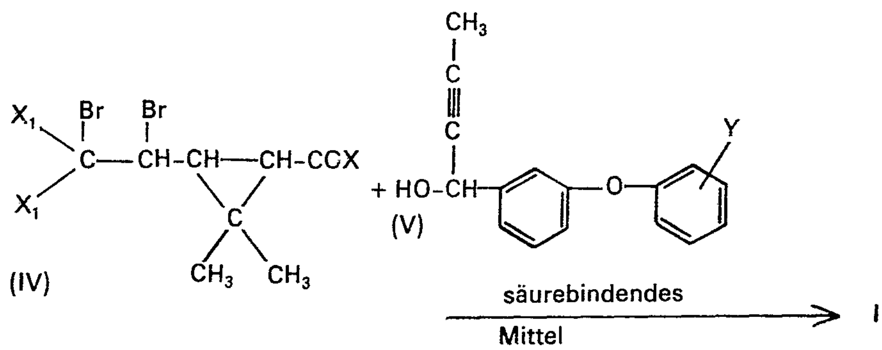


3) 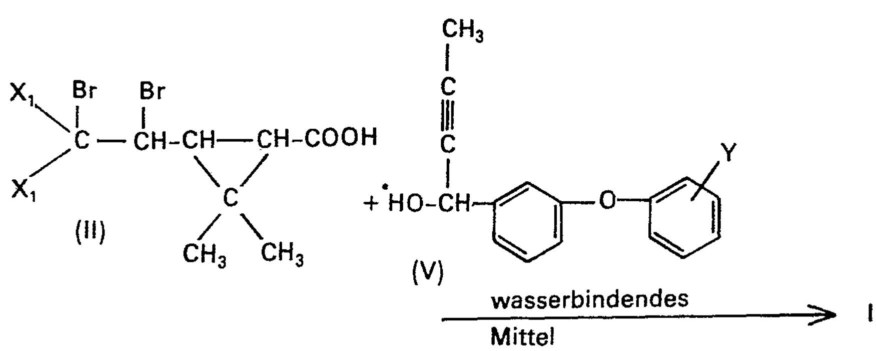

4)

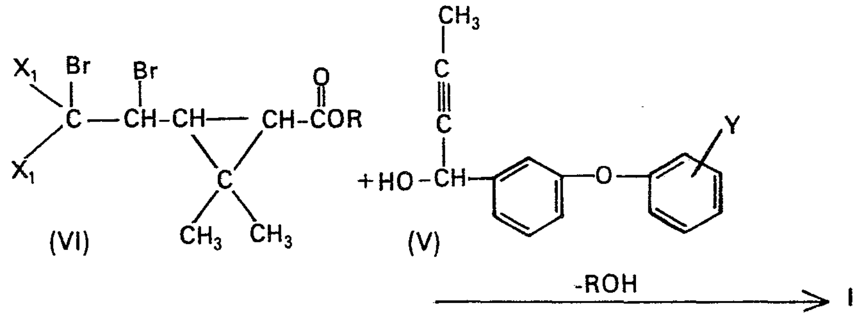


(VI) (V)

5)

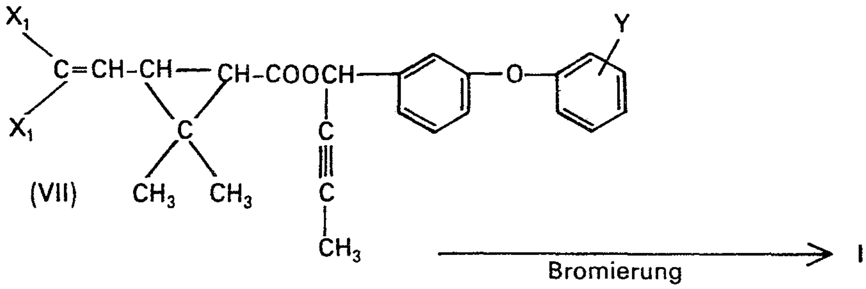


(VII)

In den Formeln II bis VII haben $X_1$ und Y die für die Formel I angegebene Bedeutung.

In den Formeln III und IV steht X für ein Halogenatom, insbesondere Chlor oder Brom, und in der Formel VI steht R für $C_1$-$C_4$-Alkyl, insbesondere für Methyl oder Äthyl. Als säurebindendes Mittel für die Verfahren 1 und 2 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate wie z.B. Kalium-t.butylat und Natriummethylat in Betracht. Als wasserbindendes Mittel für das Verfahren 3 kann z.B. Dicyclohexylcarbodiimid verwendet werden. Die Verfahren 1 bis 4 werden bei einer Reaktionstemperatur zwischen −10 und 120°C, meist zwischen 20 und 80°C bei normalem oder erhöhtem Druck und vorzugsweise in einem inerten Lösungs- oder Verdünnungsmittel durchgeführt. Als Lösungs- oder Verdünnungsmittel eignen sich z.B. Äther und ätherartige Verbindungen wie Diäthyläther, Dipropyläther, Dioxan, Dimethoxyäthan und Tetrahydrofuran; Amide wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Ausgangsstoffe der Formeln II bis VII sind bekannt oder können analog bekannten Methoden hergestellt werden.

Die Verbindungen der Formel I liegen als Gemisch von verschiedenen optisch aktiven Isomeren vor, wenn bei der Herstellung nicht einheitlich optisch aktive Ausgangsmaterialien verwendet wurden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen tierischen und pflanzlichen Schädlingen.

Insbesondere eignen sich die Verbindungen der Formel I zur Bekämpfung von Insekten, phytopathogenen Milben und von Zecken z.B. der Ordnungen Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. gegen Spodoptera littoralis und Heliothis virescens) und Gemüsekulturen (z.B. gegen Leptinotarsa decemlineata und Myzus persicae).

Wirkstoffe der Formel I zeigen auch eine sehr günstige Wirkung gegen Fliegen wie z.B. Musca domestica und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe, andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propinyläther, Propinyloxime, Propinylcarbamate

und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Verbindungen der Formel I können für sich allein oder zusammen mit geeigneten Trägern und/oder Zuschlagstoffen eingesetzt werden. Geeignete Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die Herstellung erfindungsgemässer Mittel erfolgt in an sich bekannter Weise durch inniges Vermischen und/oder Vermahlen der Wirkstoffe der Formel I mit den geeigneten Trägerstoffen, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden:

Feste Aufarbeitungsformen:

Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;

b) Lösungen

Der Gehalt an Wirkstoff in den oben beschriebenen Mitteln liegt zwischen 0,1 bis 95%, dabei ist zu erwähnen, dass bei der Applikation aus dem Flugzeug oder mittels anderer geeigneter Applikationsgeräte Konzentrationen bis zu 99,5% oder sogar reiner Wirkstoff eingesetzt werden können. Die Wirkstoffe der Formel I können beispielsweise wie folgt formuliert werden (Teile bedeuten Gewichtsteile):

Stäubemittel: Zur Herstellung eines a) 5%igen und b) 2%igen Stäubemittels werden die folgenden Stoffe verwendet:

a) 5 Teile Wirkstoff
95 Teile Talkum;

b) 2 Teile Wirkstoff
1 Teil hochdisperse Kieselsäure
97 Teile Talkum.

Der Wirkstoff wird mit den Trägerstoffen vermischt und vermahlen.

Granulat: Zur Herstellung eines 5%igen Granulates werden die folgenden Stoffe verwendet:

5 Teile Wirkstoff
0,25 Teile Epichlorhydrin
0,25 Teile Cetylpolyglykoläther
3,50 Teile Polyäthylenglykol
91 Teile Kaolin (Korngrösse 0,3–0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen, d) 10%igen Spritzpulvers werden folgende Bestandteile verwendet:

a) 40 Teile Wirkstoff
5 Teile Ligninsulfonsäure-Natriumsalz
1 Teil Dibutylnaphthalinsulfonsäure-Natriumsalz
54 Teile Kieselsäure;

b) 25 Teile Wirkstoff
4,5 Teile Calcium-Ligninsulfonat
1,9 Teile Champagne-Kreide/Hydroxyäthylcellulose-Gemisch (1:1)
1,5 Teile Natrium-dibutyl-naphthalinsulfonat
19,5 Teile Kieselsäure
19,5 Teile Champagne-Kreide
28,1 Teile Kaolin;

c) 25 Teile Wirkstoff
2,5 Teile Isooctylphenoxy-polyäthylen-äthanol
1,7 Teile Champagne-Kreide/Hydroxyäthylcellulose-Gemisch (1:1)
8,3 Teile Natriumaluminiumsilikat
16,5 Teile Kieselgur
46 Teile Kaolin;

d) 10 Teile Wirkstoff
3 Teile Gemisch der Natriumsalze von gesättigten Fettalkoholsulfaten
5 Teile Naphthalinsulfonsäure/Formaldehyd-Kondensat
82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate:

Zur Herstellung eines a) 10%igen, b) 25%igen und c) 50%igen emulgierbaren Konzentrates werden folgende Stoffe verwendet:

a) 10 Teile Wirkstoff
3,4 Teile epoxydiertes Pflanzenöl
3,4 Teile eines Kombinationsemulgators, bestehend aus Fettalkoholpolyglykoläther und Alkylarylsulfonat-Calcium-Salz
40 Teile Dimethylformamid
43,2 Teile Xylol;

b) 25 Teile Wirkstoff
2,5 Teile epoxydiertes Pflanzenöl
10 Teile eines Alkylarylsulfonat/Fettalkoholpolyglykoläther-Gemisches
5 Teile Dimethylformamid
57,5 Teile Xylol;

c) 50 Teile Wirkstoff
4,2 Teile Tributylphenol-Polyglykoläther
5,8 Teile Calcium-Dodecylbenzolsulfonat
20 Teile Cyclohexanon
20 Teile Xylol.

Aus solchen Konzentraten können durch Verdünnen mit Wasser Emulsionen jeder gewünschten Konzentration hergestellt werden.

Sprühmittel: Zur Herstellung eines a) 5%igen und b) 95%igen Sprühmittels werden die folgenden Bestandteile verwendet:

a) 5 Teile Wirkstoff
1 Teil Epichlorhydrin
94 Teile Benzin (Siedegrenzen 160-190°C);
b) 95 Teile Wirkstoff
5 Teile Epichlorhydrin.

Beispiel 1

a) Herstellung von α-Prop-1-ynyl-3-phenoxybenzylalkohol.

Zu einer Lösung von 8 g Methylacetylen in 100 ml Tetrahydrofuran wird bei 0°C eine frisch aus 4 g Magnesium, 20 g Äthylbromid in 20 ml Tetrahydrofuran hergestellte Grignardlösung langsam zugegeben und während 15 Minuten unter Argon gerührt. Zu diesem Gemisch wird eine Lösung von 27 g o-Phenoxybenzaldehyd in 100 ml Tetrahydrofuran bei 0 bis 5°C zugetropft. Nach 14stündigem Rühren bei Raumtemperatur wird das Reaktionsgemisch mit 50 g Eis auf 0°C abgekühlt, langsam mit 25 ml conc. Salzsäure versetzt und mit Äther extrahiert. Der Äther-Extrakt wird zweimal mit Wasser und zweimal mit gesättigter Kochsalzlösung gewaschen, über Natriumsulfat getrocknet, filtriert und eingeengt.

Das Produkt wird mit Essigester/Hexan (1:4) als Eluiermittel über Silikagel chromatographiert.

Man erhält die Verbindung der Formel

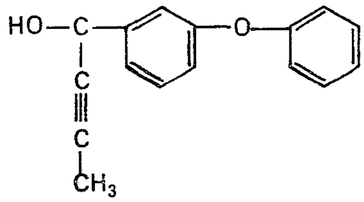

mit einem Brechungsindex von $n_D^{20°} = 1,5898$.

b) Herstellung von α-Prop-1-ynyl-3-phenoxy benzyl-2,2-dimethyl-3-(-1,2-dibrom-2,2-dichloräthyl)-cyclopropan-1-carboxylat.

Zu einer Lösung von 5,64 g 2,2-Dimethyl-3-(1,2-dibrom-2,2-dichloräthyl)-cyclopropankarbonsäurechlorid und 1,5 ml Pyridin in 40 ml Toluol wird bei 0°C eine Lösung von 3,5 g α-Prop-1-ynyl-3-phenoxybenzylalkohol in 20 ml Toluol zugetropft. Das Reaktionsgemisch wird 14 Stunden bei Raumtemperatur gerührt, dann mit Äther verdünnt, einmal mit Wasser, einmal mit 2n-Salzsäure, dreimal mit gesättigter Kochsalzlösung gewaschen, über Natriumsulfat getrocknet, filtriert und eingeengt. Das Produkt wird mit Äther/Hexan (1:3) als Eluiermittel über Silikagel chromatographiert.

Man erhält die Verbindung der Formel

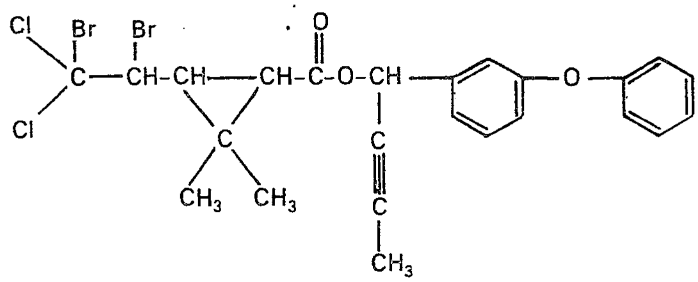

als ein diastereomeres Gemisch mit einem Brechungsindex von $n_D^{20°} = 1,5879$.

Auf analoge Weise werden auch folgende Verbindungen hergestellt:

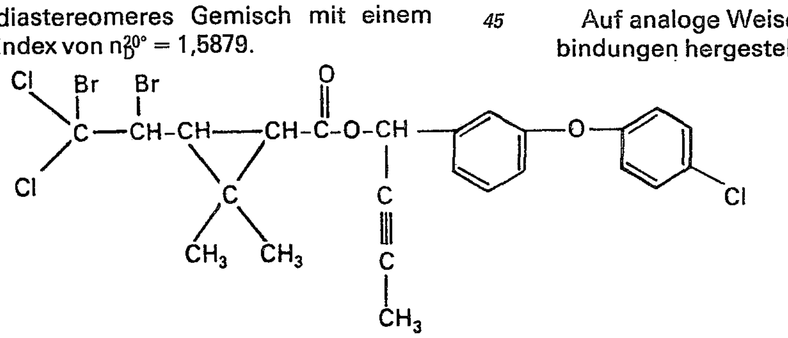

$n_D^{20°} = 1,5872$

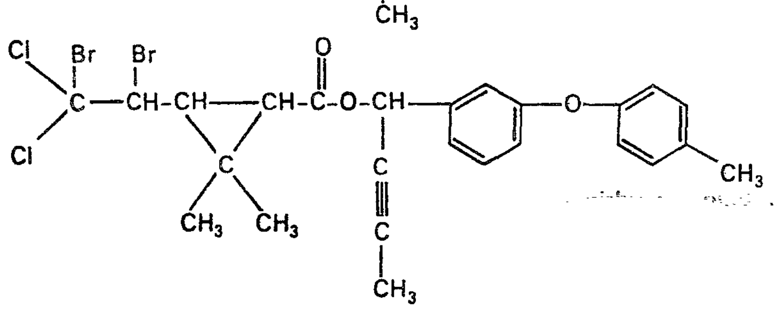

$n_D^{20°} = 1,5803$

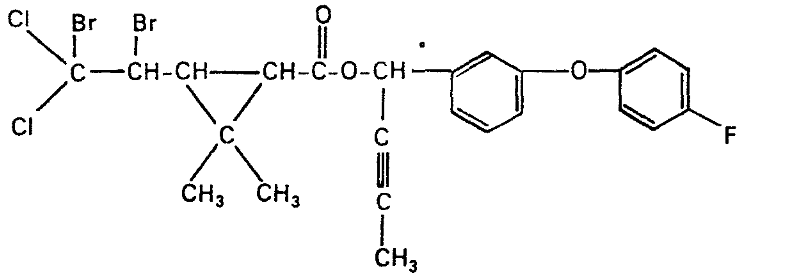

$n_D^{20°} = 1,5712$

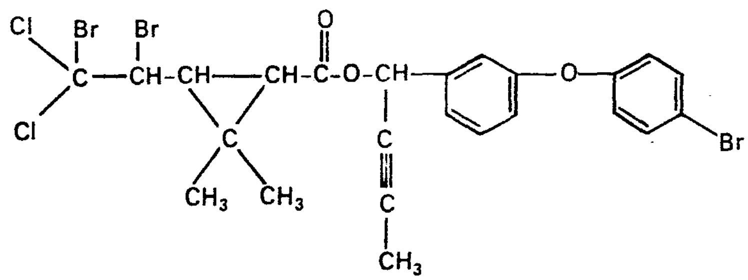

$n_D^{20°}$ — 1,5981

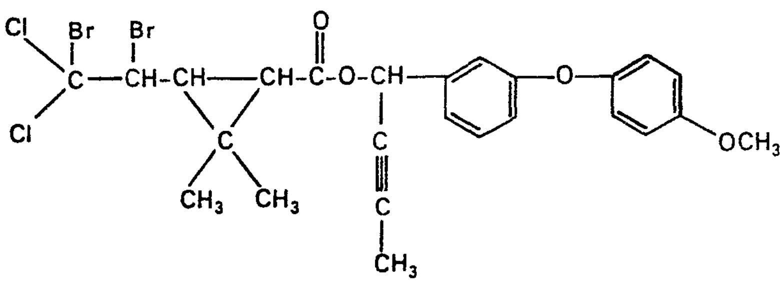

Harz

Beispiel 2

A) Insektizide Frassgift-Wirkung

Baumwollpflanzen wurden mit einer 0,05%igen wässrigen Wirkstoffemulsion (erhalten aus einem 10%igen emulgierbaren Konzentrat) besprüht.

Nach dem Antrocknen des Belages wurden die Baumwollpflanzen je mit Spodoptera littoralis- und Heliothis virescens-Larven $L_3$ besetzt. Der Versuch wurde bei 24°C und 60% relativer Luftfeuchtigkeit durchgeführt.

Verbindungen gemäss Beispiel 1 zeigten im obigen Test eine gute insektizide Frassgift-Wirkung gegen Spodoptera- und Heliothis-Larven.

Beispiel 3

Akarizide Wirkung

Phaseolus vulgaris Pflanzen wurden 12 Stunden vor dem Test auf akarizide Wirkung mit einem infestierten Blattstück aus einer Massenzucht von Tetranychus urticae belegt. Die übergelaufenen beweglichen Stadien wurden aus einem Chromatographiezerstäuber mit den emulgierten Testpräparaten derart besprüht, dass kein Ablaufen der Spritzbrühe eintrat. Nach zwei und 7 Tagen wurden Larven, Adulte und Eier unter dem Binokular auf lebende und tote Individuen ausgewertet und das Ergebnis in Prozenten ausgedrückt. Während der «Haltezeit» standen die behandelten Pflanzen in Gewächshauskabinen bei 25°C.

Verbindungen gemäss Beispiel 1 wirkten im obigen Test gegen Adulte, Larven und Eier von Tetranychus urticae.

Beispiel 4

Wirkung gegen Zecken

A) Rhipicephalus bursa

Je 5 adulte Zecken bzw. 50 Zeckenlarven wurden in ein Glasröhrchen gezählt und für 1 bis 2 Minuten in 2 ml einer wässrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

B) Boophilus microplus (Larven)

Mit einer analogen Verdünnungsreihe wie beim Test A) wurden mit je 20 sensiblen resp. OPresistenten Larven Versuche durchgeführt. (Die Resistenz bezieht sich auf die Verträglichkeit von Diazinon). Verbindungen gemäss Beispiel 1 wirkten in diesen Tests gegen Adulte und Larven von Rhipicephalus bursa und sensible resp. OP-resistente Larven von Boophilus microplus.

Beispiel 5

a) Chemische Reinigung

Es wurde eine 10%-Lösung einer Verbindung gemäss Formel I in Methylcellosolve hergestellt. Ein Volumenteil dieser Lösung wurde mit 200 Volumenteilen eines zur Trockenreinigung geeigneten Lösungsmittels, z.B. eine passende Benzinfraktion oder Perchloräthylen, verdünnt. Gewünschtenfalls können noch reinigungsfördernde Zusätze beigefügt werden. Die Wollartikel wurden nun wie üblich mit dieser Reinigungsflüssigkeit behandelt und anschliessend auf einen Lösungsmittelgehalt von ca. 100% des Wollgewichtes abgeschleudert. Sie erwiesen sich nach dem Trocknen als Motten- und Käfer-echt. In analoger Weise können gleich oder ähnlich zusammengesetzte Bäder auch zur Motten- und Käferausrüstung unbehandelter oder bereits sonstwie behandelter oder gereinigter Artikel verwendet werden.

Auch zum Besprühen oder Bespritzen von Wolle in jedem Verarbeitungszustand können ähnliche Gemische herangezogen werden.

b) Sprühen

Es wurde eine 0,5%-Lösung eines Wirkstoffes der Formel I in einem leichtflüchtigen organischen Lösungsmittel angesetzt. Der Wollartikel wurde mit einer üblichen Sprüheinrichtung besprüht, so dass 2 × 15 g/m² Wirkstofflösung appliziert wurden. Bei einem Ausnützungseffekt des Aerosols von 30% sind dann etwa 400 ppm an Wirkstoff auf dem Material. Die so ausgerüsteten Wollgewebe erwiesen sich als motten- und käferecht.

c) Foulardmethode

Es wurde eine 0,4% (Gewicht/Volumen) Stammlösung des zu prüfenden Wirkstoffes in Methylcellosolve bereitet. 12,5 ml Stammlösung wurden mit Methylcellosolve, welche 0,65 g/l eines anionische, sulfonierten Öles enthält, auf 50 ml verdünnt – Lösung Nr. 1. 25 ml Lösung Nr. 1 wurden mit Methylcellosolve, welche 0,5 g/l eines anionischen, sulfonierten Öles enthält, auf 50 ml verdünnt – Lösung Nr. 2. 25 ml von Lösung Nr. 2 wurden erneut mit Methylcellosolve, welche 0,5 g/l eines anionischen, sulfonierten Öles enthält, auf 50 ml verdünnt (Lösung Nr. 3).

Von den Lösungen Nr. 1, 2 und 3 wurden je 3 ml in Kristallisierschalen geleert und je eine geköderte Rondelle aus Wollflanell 3 Sekunden darin benetzt. Die feuchten Rondellen wurden anschliessend zwischen Aluminiumfolien foulardiert und zwar derart, dass die abgequetschten Rondellen je 50% (Volumen/Gewicht) Flotte aufgenommen haben. Die Konzentrationen an Wirkstoff sind dann der Reihe nach 500 ppm, 250 ppm und 125 ppm für behandelte Rondellen aus den Lösungen Nr. 1, 2 und 3.

Die feuchten Rondellen wurden an der Luft getrocknet und den gleichen biologischen Prüfungen unterworfen wie bei der Färbebadmethode.

Substanzen gemäss Formel I zeigten eine sehr gute Wirkung bis 125 ppm gegen Tineola bisselliela und Tinea pellionella.

d) Färbemethode

Es wurde eine 0,4% (Gewicht/Volumen) Stammlösung des zu prüfenden Wirkstoffes der Formel I in Methylcellosolve bereitet. Dann wurde bei Zimmertemperatur eine wässrige Applikationsflotte hergestellt, die auf 120 ml destilliertes Wasser 0,12 ml Sandozin KB, 0,6 ml Ameisensäure 1/10 und 0,75 ml der 0,4% Stammlösung enthielt. Dann wurden 3 g Wollflanell-Gewebe mit heissem Wasser durchnetzt und bei Zimmertemperatur eingegeben. Unter ständigem Umziehen des Wollmusters wurde die Badtemperatur innert 20 Minuten auf 60°C erwärmt und 30 Minuten bei 60°C behandelt. Dann wurde abgekühlt, das Wollmuster zweimal 3 Minuten bei destilliertem Wasser gespült, von Hand abgequetscht und an der Luft getrocknet. Die Wirkstoffkonzentration beträgt 1000 ppm, berechnet auf das Wollgewicht.

Das so getrocknete Muster wurde der Mottenechtheitsprüfung (Frasschutz gegen Kleidermotte Tineola biselliella H.), gemäss der Vorschrift des schweizerischen Normenverbandes SNV-195901, sowie der Echtheitsprüfung gegen Larven des Pelzkäfers (Attagenas piceus oliv. [black]) und Teppichkäfers (Anthrenus vorax Waterhouse), gemäss SNC 195902, unterworfen.

Es wurden je 10 Larven von Anthrenus vorax Waterhouse und je 10 6 bis 7 Wochen alte Larven von Attagenus piceus oliv. (black) zur Prüfung verwendet. Aus den behandelten Wollflanellmustern wurden Stücke gleicher Grösse ausgeschnitten und 14 Tage lang bei konstanter Temperatur (28°C) und konstanter relativer Luftfeuchtigkeit (65%) dem Angriff (Frass) von je 15 Larven des entsprechenden Schädlings ausgesetzt. Die Beurteilung erfolgte einerseits nach dem relativen Gewichtsverlust des Prüflings und andererseits nach der Anzahl noch lebender Organismen.

Die Substanzen gemäss Formel I zeigten eine sehr gute Wirkung gegen die 3 verwendeten Schädlinge.

**Patentansprüche**

1. Ein Cyclopropankarbonsäureester der Formel

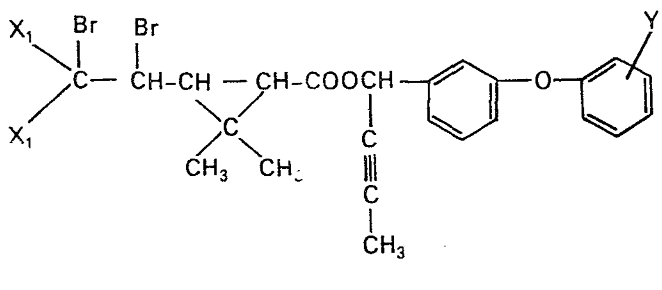

worin $X_1$ Fluor, Chlor oder Brom und Y Wasserstoff, Methyl, Fluor, Chlor, Brom oder Jod bedeuten.

2. Eine Verbindung gemäss Anspruch 1, worin

$X_1$ Fluor, Chlor oder Brom und Y Wasserstoff, p-Methyl, p-Fluor, p-Chlor oder p-Brom bedeuten.

3. Eine Verbindung gemäss Anspruch 2, worin $X_1$ Fluor, Chlor oder Brom und Y Wasserstoff bedeuten.

4. Die Verbindung gemäss Anspruch 3 der Formel

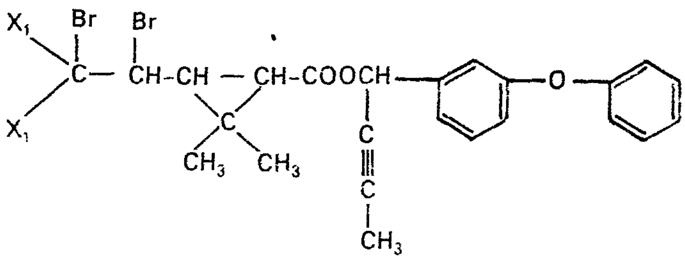

5. Verfahren zur Herstellung einer Verbindung gemäss Anspruch 1, dadurch gekennzeichnet, dass man eine Verbindung der Formel

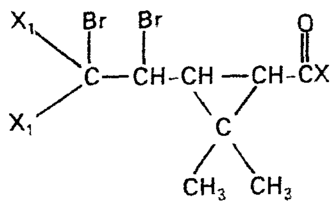

in Gegenwart eines säurebindenden Mittels mit einer Verbindung der Formel

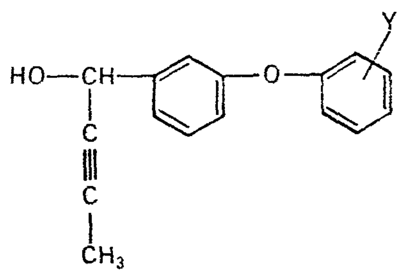

umsetzt, worin $X_1$ und Y die im Anspruch 1 angegebene Bedeutung haben und X für ein Halogenatom steht.

6. Ein Schädlingsbekämpfungsmittel, welches als aktive Komponente eine Verbindung gemäss Anspruch 1 und geeignete Träger- und/oder andere Zuschlagstoffe enthält.

7. Verwendung einer Verbindung gemäss Anspruch 1 zur Bekämpfung von verschiedenartigen tierischen und pflanzlichen Schädlingen.

8. Verwendung gemäss Anspruch 7 zur Bekämpfung von Insekten und Vertretern der Ordnung Akarina.

9. Verwendung gemäss Anspruch 7 zur Bekämpfung von keratinfressenden Insekten.

**Claims**

1. A cyclopropanecarboxylic acid ester of the formula

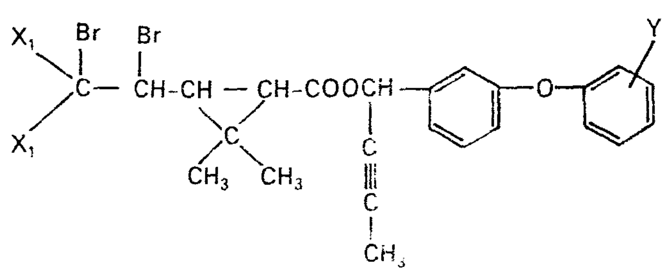

in which $X_1$ is fluorine, chlorine or bromine, and Y is hydrogen, methyl, fluorine, chlorine, bromine or iodine.

2. A compound according to Claim 1 wherein $X_1$ is fluorine, chlorine or bromine, and Y is hydrogen, p-methyl, p-fluorine, p-chlorine or p-bromine.

3. A compound according to Claim 2 wherein $X_1$ is fluorine, chlorine or bromine, and Y is hydrogen.

4. The compound according to Claim 3 of the formula

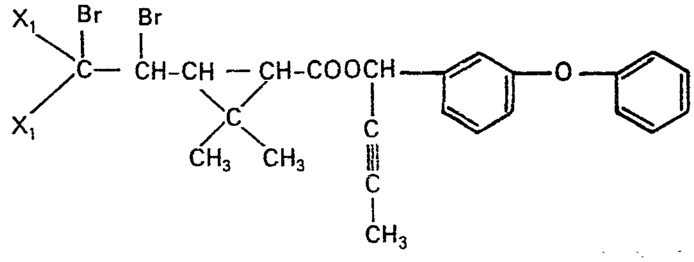

5. A process for producing a compound according to Claim 1, which process comprises reacting a compound of the formula

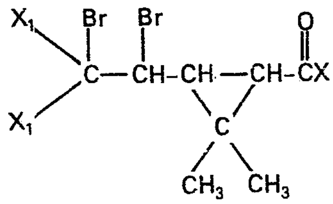

in the presence of an acid-binding agent, with a compound of the formula

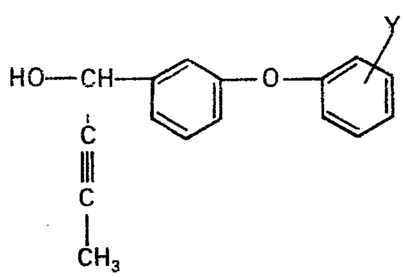

in which $X_1$ and Y have the meanings given in Claim 1, and X is a halogen atom.

6. A pesticidal composition which comprises a compound according to Claim 1 as active ingredient, and suitable carriers and/or other additives.

7. Use of a compound according to Claim 1 for controlling various animal and plant pests.

8. Use according to Claim 7 for controlling insects, and representatives of the order Acarina.

9. Use according to Claim 7 for controlling keratin-eating insects.

**Revendications**

1. Un ester cyclopropane-carboxylique de formule

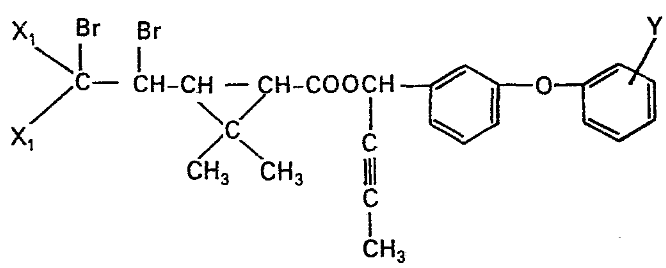

dans laquelle $X_1$ représente le fluor, le chlore ou le brome, et Y représente l'hydrogène, un groupe méthyle, le fluor, le chlore, le brome ou l'iode.

2. Un composé selon la revendication 1, dans lequel $X_1$ représente le fluor, le chlore ou le brome, et Y représente l'hydrogène, un groupe p-méthyle, un atome de fluor en position para, un atome de chlore en position para ou un atome de brome en position para.

3. Un composé selon la revendication 2, dans lequel $X_1$ représente le fluor, le chlore ou le brome, et Y représente l'hydrogène.

4. Le composé selon la revendication 3, de formule

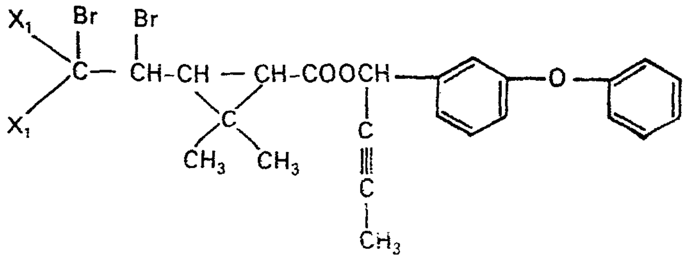

5. Procédé de préparation d'un composé selon la revendication 1, caractérisé en ce que l'on fait réagir un composé de formule

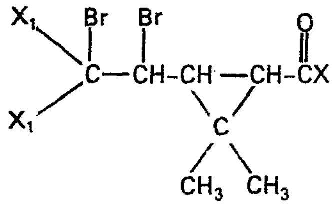

en présence d'un agent fixant les acides, avec un composé de formule

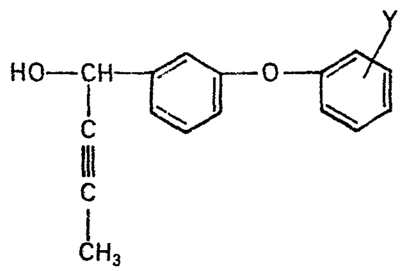

$X_1$ et Y ayant les significations indiquées dans la revendication 1 et X représentant un atome d'halogène.

6. Un produit pesticide contenant en tant que composant actif un composé selon la revendication 1 et des véhicules et/ou autres additifs appropriés.

7. Utilisation d'un composé selon la revendication 1 pour la lutte contre les parasites animaux et végétaux de types variés.

8. Utilisation selon la revendication 7, pour la lutte contre les insectes et les représentants de l'ordre des acariens.

9. Utilisation selon la revendication 7, pour la lutte contre les insectes dévorant la kératine.